# Europäisches Patentamt

# European Patent Office

# Office européen des brevets

(11) Veröffentlichungsnummer: **0 016 239**
**B1**

(12) ## EUROPÄISCHE PATENTSCHRIFT

(45) Veröffentlichungstag der Patentschrift:
**30.12.81**

(51) Int. Cl.³: **A 61 K 7/06, A 61 K 7/48**

(21) Anmeldenummer: **79100912.9**

(22) Anmeldetag: **27.03.79**

(54) **Kosmetisches Haar- und Hautpflegemittel und dessen Herstellung.**

(43) Veröffentlichungstag der Anmeldung:
**01.10.80 Patentblatt 80/20**

(45) Bekanntmachung des Hinweises auf die Patenterteilung:
**30.12.81 Patentblatt 81/52**

(84) Benannte Vertragsstaaten:
**BE FR GB LU NL**

(56) Entgegenhaltungen:
**DE-A-2 757 024**
**CHEMICAL ABSTRACTS, Band 76, Nr. 1,**
**03-01-1972, Seite 39,**
**Zusammenfassung 355p**
**und 9th Collective Chemical**
**Substance Index 1972–1976,**
**Seite 30535 CS**
**Columbus, Ohio US**
**A. CAPEK et al.:**
**«Antimicrobial agents. IX.**
**Effect for steroids on dermatophytes»**
**Proc. Soc. Exper. Biol & Med.,**
**82 (1953), 243–247**

(73) Patentinhaber: **Tamm, Jürgen, Prof. Dr. med.,**
**Schrammsweg 8, D-2000 Hamburg 20 (DE)**

(72) Erfinder: **Tamm, Jürgen, Prof. Dr. med., Schrammsweg 8,**
**D-2000 Hamburg 20 (DE)**

(74) Vertreter: **Freiherr von Uexküll, Jurgen-Detlev et al,**
**Uexküll & Stolberg Patentanwälte Beselerstrasse 4,**
**D-2000 Hamburg 52 (DE)**

ACTORUM AG.

## Kosmetisches Haar- und Hautpflegemittel und dessen Herstellung

Die Erfindung betrifft ein kosmetisches Haar- und Hautpflegemittel, das zur Erzielung und Erhaltung eines kräftigen Haarwuchses und einer gesunden und gepflegten Haut, insbesondere Kopfhaut, hervorragend geeignet ist sowie dessen Herstellung.

Es ist seit langem bekannt, dass kosmetische Störungen des Haares und der Haut häufig auf Verschiebungen im Hormongleichgewicht zurückzuführen sind. Man hat daher Versuche unternommen, diese kosmetischen Störungen, wie übermässigen Haarausfall oder das Auftreten von Akne, durch Behandlungen mit kosmetischen Präparaten, denen gewisse Hormone und/oder Vitamine zugesetzt sind, zu beseitigen. So wurde festgestellt, dass eine Reihe natürlicher synthetischer weiblicher Sexualhormone (Östron, Östradiol, Stilbestrol) in ihrer hormonalen Wirkung und in ihrer Wirkung auf das Hautgewebe einander ähnlich sind, siehe z.B. J.S. Jellinek, Kosmetologie, Heidelberg (1965), S. 409/410. Es lag daher nahe, die Wirkung der Sexualhormone auch kosmetisch auszunutzen.

Die Behandlung mit Hormoncremes, beispielsweise mit östrogenhaltigen Cremes, führt gerade bei alternder Haut zu einem frischen, verjüngend wirkenden Erscheinungsbild, siehe J.S. Jellinek, Kosmetologie, (1965), S. 423. In gewissen biologischen Haarwässern werden Östrogene und östrogenhaltige Extrakte verarbeitet, da man animmt, dass diese weiblichen Sexualhormone den Haarwuchs anregen, siehe J.S. Jellinek a.a.O., S. 454.

Da solche Präparate hauptsächlich von Männern verwendet werden, die in der Regel wesentlich häufiger vom Haarausfall betroffen sind als Frauen, ist die Anwendung solcher kosmetischer Mittel nicht problemlos. Hormone sind bekanntlich bereits in ausserordentlich geringen Mengen wirksam und entfalten ihre Wirkung in der Regel nicht nur lokal an der aufgebrachten Stelle, sondern dringen in die tieferen Hautschichten ein, wo sie zum Teil in die Blutbahn gelangen. Schon sehr geringe Mengen eines Hormons im Blut vermögen aber deutliche körperliche und auch seelische Wirkungen hervorzurufen. Bei der Anwendung hormonaler Wirkstoffe in kosmetischen Präparaten kann es daher zu unerwünschten systematischen Effekten kommen, ein Nachteil, der ihre Verwendung für kosmetische Zwecke ausserordentlich einschränkt.

Um diese nachteiligen Wirkungen weitgehend zu vermeiden, hat man nach Wirkstoffen gesucht, die zwar die gewünschten kosmetischen Wirkungen besitzen, deren hormonale Wirksamkeit aber nur gering ist. Als den Östrogenen chemisch nahe verwandte Wirkstoffe sind z.B. Progesteron sowie eine Reihe ähnlicher Steroide bekannt geworden, siehe DE-OS 1 617 857. Sie besitzen jedoch immer eine Wirkung als Sexualhormone, so dass auch ihrer Verwendung in Kosmetik-Präparaten enge Grenzen gesetzt sind.

Es ist ferner bekannt, dass die androgenetisch bedingten kosmetischen Störungen, beispielsweise die androgenetische Alopezie, massgeblich durch das männliche Keimdrüsenhormon Testosteron ausgelöst werden. So geht der Haarausfall im Bereich des Stirn- und Scheitelhaares mit dem Erreichen einer gewissen kritischen Höhe der Testosteron-Konzentration im Blut einher. Bei der Frau genügen häufig relativ geringfügige Überschreitungen ihres normalen Plasmaspiegels an Testosteron, um einen übermässigen Haarwuchs, insbesondere Barthaarwuchs (Hirsutismus), auszulösen.

Den gegensätzlichen Wirkungen des Testosterons, nämlich einerseits der Förderung des Haarausfalls im Bereich der Stirn- und Scheitelhaare, andererseits der Förderung des Bartwuchses, liegen die gleichen Mechanismen zugrunde. Diese sind weitgehend aufgeklärt und haben dazu geführt, dass gezielte Versuche unternommen wurden, um durch entsprechende Beeinflussung dieses Mechanismus die oben genannten Störungen zu mindern oder zu beseitigen. So wurde z.B. das Gelbkörperhormon Progesteron eingesetzt, um die Wirkung des Enzyms 5α-Reductase, durch welches Testosteron in der Zelle zu Dihydrotestosteron reduziert wird, zu hemmen, siehe Mauvais-Jarvis, Kuttenn u. Wright, Ann. d. Endocrinologie 36 (1975), S. 55. Dihydrotestosteron ist deutlich wirksamer als Testosteron und wird mit einem spezifischen Eiweisskörper (Rezeptor) gekoppelt. Dieser Komplex wird in den Zellkern transportiert, wo er auf den genetischen Code einwirken kann. Man hat daher auch schon versucht, die Bildung des Rezeptorkomplexes zu unterbinden, wobei sich als geeigneter Wirkstoff Cyproteronacetat erwies. Obwohl man auf diese Weise bei lokaler Anwendung von Progesteron ein Nachlassen des Haarausfalls bei Männern mit androgenetischer Alopezie beobachtet hat, ist die Verwendung der genannten Wirkstoffe in rein kosmetischen Präparaten ebenfalls problematisch, da auch diese Wirkstoffe eine eigene Hormonwirkung haben, oder unbefriedigend, da sie z.T. lokal nicht wirksam sind.

Mit der Erfindung, wie sie in den Ansprüchen gekennzeichnet ist, wird die Aufgabe gelöst, lokal anwendbare kosmetische Mittel für die Haar- und Hautpflege zu schaffen, die zur Bekämpfung der vorstehend genannten kosmetischen Störungen geeignet sind, deren Wirkstoff aber keine Hormonwirkung hat und auch bei länger andauernder lokaler Applikation keine unerwünschten Nebenwirkungen zeigt.

Der erfindungsgemäss eingesetzte Wirkstoff 11α-Hydroxyprogesteron ist bekannt. Er wurde bereits im Jahre 1952 synthetisch hergestellt und zeigt mit den hohen Dosen des Tierexperiments keine nennenswerten Hormonwirkungen, siehe Proc. Soc. Exper. Biol. & Med. 82, (1953), Seiten 243–247. 11α-Hydroxyprogesteron kommt nicht im menschlichen Organismus vor und besitzt eine spezifische Hemmwirkung gegen die 5α-Reductase des Testosterons.

Das erfindungsgemäss eingesetzte 11α-Hydro-

xyprogesteron und seine pharmazeutisch wirksamen und verträglichen Ester eignen sich in hervorragendem Masse zur Behandlung der oben genannten kosmetischen Störungen, wobei diese Wirkstoffe gleichzeitig den Vorteil besitzen, keine hormonalen oder anderen unerwünschten Nebenwirkungen zu zeigen, so dass sie unbedenklich in kosmetischen Präparaten eingesetzt werden können. Insbesondere eignen sich die diese Wirkstoffe enthaltenden erfindungsgemässen kosmetischen Mittel zur lokalen Haar- und Hautpflege, wobei sie bereits nach relativ kurzer Behandlungszeit (8 bis 12 Tage) einen erheblichen Rückgang des Fettens bei überfettetem Haar sowie nach entsprechend längerer Applikation einen deutlichen Rückgang des Haarausfalls bewirken. Auch bei der Behandlung von Akne wird bereits nach bemerkenswert kurzer Behandlungsdauer ein Zurückgehen und in vielen Fällen ein Verschwinden der betreffenden Symptome erreicht.

Die vorteilhaften kosmetischen Wirkungen des 11α-Hydroxyprogesterons bei der lokalen Behandlung von Haarstörungen, wie androgenetischer Alopezie, übermässigem Haarfetten sowie von Akne vulgaris, wurden durch sich über mehrere Monate erstreckende klinische Versuche an Männern und Frauen im Alter zwischen 16 und 54 Jahren belegt.

So wurden 10 Männer, die an übermässigem Haarfetten und Kopfhaarausfall litten, über einen Zeitraum von 4 bis 6 Monaten mit einer 1%igen Lösung von 11α-Hydroxyprogesteron in 60%igem Ethanol lokal behandelt, und zwar wurden täglich etwa 10 bis 15 ml der Lösung in die Kopfhaut einmassiert. Ausnahmslos alle Probanden spürten bereits 8 bis 12 Tage nach Beginn der Behandlung einen erheblichen Rückgang des lästigen Haarfettens. Nach etwa 5 bis 6 Wochen wurde auch ein deutlicher Rückgang des Haarausfalls beobachtet, der sich dann während der weiteren Behandlung völlig normalisierte. Nebenerscheinungen irgendwelcher Art wurden während der gesamten Behandlungszeit nicht beobachtet.

In einem weiteren Versuch unterzogen sich 14 Frauen, die ebenfalls unter starkem Haarfetten und bzw. oder androgenetischer Alopezie litten, der oben geschilderten Behandlung. Hier stellten sich bei 12 Versuchspersonen innerhalb von 8 bis 12 Tagen die ersten vorteilhaften Wirkungen in Form einer deutlichen Verminderung des Haarfettens ein, und nach etwa 5 bis 6 Wochen liess sich ein deutlicher Rückgang des Haarausfalls feststellen. Allerdings empfanden einige der Probanden die sich einstellende Trockenheit des Haares als unangenehm. Durch gewisse hautpflegende Zusätze und/oder weitere Verminderung des Alkoholgehaltes oder Verwendung eines anderen geeigneten Alkohols, z.B. Isopropanol, lassen sich diese Begleiterscheinungen, die nur gelegentlich und bei Personen mit besonders empfindlicher Haut auftreten, weitgehend zurückdrängen. Ansonsten wurden auch bei den Frauen keinerlei Nebenwirkungen lokaler oder allgemeiner Art während der Behandlung beobachtet.

In einer weiteren Untersuchung wurden 5 Frauen und 4 Männer wegen Akne vulgaris im Gesicht und am oberen Körperstamm mit einer spezifischen Nachtcreme behandelt, die in einer üblichen fetthaltigen O/W-Emulsion etwa 1,4 Gew.-% 11α-Hydroxyprogesteron enthielt. Die befallenen Hautstellen wurden mit dieser Creme täglich einmal eingerieben. In allen Fällen wurde bereits nach 8 bis 10 Tagen ein merklicher Rückgang der Akne-Effloreszenzen beobachtet. Die weitere lokale Behandlung brachte nach etwa 15 bis 20 Tagen eine nachhaltige Verbesserung des Krankheitsbildes. Während und nach der Behandlung wurden keinerlei lokale oder allgemeine Nebenerscheinungen festgestellt.

In weiteren Paralleluntersuchungen wurde auch der Essigsäureester des 11α-Hydroxyprogesterons sowohl in 1%iger alkoholischer Lösung als auch in 1,4%iger Menge in einer Nachtcreme an männlichen und weiblichen Probanden angewandt, die unter starkem Haarfetten, Kopfhaarausfall und Akne vulgaris litten. In allen Fällen wurden die gleichen Erfolge erzielt wie mit 11α-Hydroxyprogesteron.

In den folgenden Tabellen sind Einzelergebnisse über die kosmetische Wirkung von 11α-Hydroxyprogesteron zusammengestellt:
Es bedeuten: + = wirksam; + + = gut wirksam; + + + = sehr gut wirksam; − = nicht wirksam; ± = Wirkung zweifelhaft.

Tabelle 1:
Behandlung von übermässigem Haarfetten und androgenetischer Alopezie mit 1%iger 11α-Hydroxyprogesteron-Lösung in 60%igem Ethanol, an Frauen.

| lfd. Nr. | Alter der Probanden (in Jahren) | Behandlung tägl. Menge | Ergebnisse | | | Nebenwirkungen |
|---|---|---|---|---|---|---|
| | | | Dauer (Monate) | Haarfetten | Alopezie | |
| 1 | 45 | 1×10 ml | 6 | + | − | Haar stumpf |
| 2 | 31 | 2×10 ml | 5 | + + | + | keine |
| 3 | 17 | 2×10 ml | 11 | + + + | + | do. |
| 4 | 36 | 2×10 ml | 11 | + + + | + + | do. |
| 5 | 40 | 1×15 ml | 6 | + + + | ± | do. |

| lfd. Nr. | Alter der Probanden (in Jahren) | Behandlung tägl. Menge | Dauer (Monate) | Ergebnisse Haarfetten | Alopezie | Nebenwirkungen |
|---|---|---|---|---|---|---|
| 6 | 40 | 1×15 ml | 5 | | + | do. |
| 7 | 33 | 1×15 ml | 6 | ++ | + | do. |
| 8 | 40 | 1×15 ml | 5 | + | + | do. |
| 9 | 43 | 2×10 ml | 12 | +++ | + | do. |
| 10 | 24 | 2×10 ml | 8 | ± | + | do. |
| 11 | 40 | 2×10 ml | 9 | ++ | + | do. |
| 12 | 27 | 1×10 ml | 6 | +++ | + | do. |
| 13 | 39 | 2×10 ml | 9 | +++ | + | do. |
| 14 | 37 | 1×15 ml | 4 | +++ | ++ | do. |
| 15 | 21 | 1×15 ml | 10 | +++ | ++ | do. |
| 16 | 44 | 1×15 ml | 4 | +++ | | do. |
| 17 | 26 | 1×15 ml | 4 | ++ | ± | do. |
| 18 | 32 | 1×10 ml | 1,5 | + | ± | do. |

Tabelle 2:
Behandlung von übermässigem Haarfetten und androgenetischer Alopezie mit 1%iger 11α-Hydroxyprogesteron-Lösung in 60%-igem Ethanol, an Männern.

| lfd. Nr. | Alter der Probanden (Jahre) | Behandlung tägl. Menge | Dauer (Monate) | Ergebnisse Haarfetten | Alopezie | Nebenwirkungen |
|---|---|---|---|---|---|---|
| 1 | 37 | 2×12 ml | 11 | +++ | + | keine |
| 2 | 24 | 1×12 ml | 11 | | + | do. |
| 3 | 54 | 1×15 ml | 6 | +++ | ++ | do. |
| 4 | 27 | 1×15 ml | 10 | ++ | + | do. |
| 5 | 25 | 1×10 ml | 11 | +++ | + | do. |
| 6 | 29 | 1×10 ml | 11 | +++ | + | do. |
| 7 | 33 | 1×15 ml | 8 | +++ | ++ | do. |
| 8 | 36 | 1×15 ml | 3 | ++ | + | do. |
| 9 | 35 | 1×15 ml | 3 | ++ | + | do. |
| 10 | 51 | 1×15 ml | 3 | +++ | ± | do. |
| 11 | 49 | 1×15 ml | 11 | +++ | ++ | do. |
| 12 | 37 | 1×15 ml | 2 | ++ | ± | do. |

Tabelle 3:
Behandlung von Akne vulgaris mit einer 1% 11α-Hydroxyprogesteron enthaltenden fetthaltigen Nachtcreme (O/W-Emulsion), die in üblicher Weise und Menge als Kosmetikum aufgetragen wurde.

| lfd. Nr. | Alter der Probanden (Jahre) | Behandlung Ort | Dauer (Monate) | Ergebnisse Akne vulgaris | Nebenwirkungen |
|---|---|---|---|---|---|
| 1 | 43 | Gesicht, | 7 | +++ | keine |
| | | oberer Stamm | 7 | +++ | do. |
| 2 | 16 | Gesicht, | 6 | ++ | do. |
| | | oberer Stamm | 6 | ++ | do. |
| 3 | 17 | Gesicht, | 6 | ++ | do. |
| | | Rücken | 6 | ++ | do. |
| 4 | 36 | Gesicht | 5 | +++ | do. |
| 5 | 33 | Gesicht | 3 | ++ | do. |

| lfd. Nr. | Alter der Probanden (Jahre) | Behandlung Ort | Dauer (Monate) | Ergebnisse Akne vulgaris | Nebenwirkungen |
|---|---|---|---|---|---|
| 6 | 24 | Gesicht | 5 | +++[a] | do. |
| 7 | 39 | Gesicht | 5 | ++ | do. |
| 8 | 38 | Gesicht, oberer Stamm | 4 4 | +++ +++ | do. do. |
| 9 | 16 | Gesicht | 1,5 | + | do. |

[a] Die unter ldf. Nr. 6 behandelte Probandin konnte nach 5-monatiger Behandlung als geheilt entlassen werden.

In einem weiteren Test wurde die Stirnhaut von zwei gesunden jungen Männern mit einer 1%igen Lösung von 11α-Hydroxyprogesteron in 60%igem Ethanol täglich einmal eingepinselt. Vor der Behandlung und jeweils am 14. und 15. Behandlungstag wurde der Sebumgehalt auf der Stirnhaut gravimetrisch nach der Methode von J.S. Strauss und P.E. Pochi, J. invest. Derm. 36, 293–298 (1961) gemessen. Die Ergebnisse sind aus Tabelle 4 ersichtlich.

Tabelle 4:
Beeinflussung des Sebumgehaltes in der Stirnhaut durch Behandlung mit 1%iger 11α-Hydroxyprogesteronlösung in 60%igem Ethanol

| Proband | Sebumgehalt in mg/10 cm$^2$/3h vor der Behandlung | | am 14. Tag | am 15. Tag |
|---|---|---|---|---|
| Blindprobe | 2,54 | | 2,65 | |
| A | 3,98 | 3,70 | 1,50 | 1,83 |
| B | 3,13 | 3,20 | 1,80 | 1,90 |

Aus der Tabelle 4 ist deutlich zu erkennen, dass die relativ kurze Behandlung mit 11α-Hydroxyprogesteron zu einer eindeutigen Senkung der Talgdrüsentätigkeit führt. Dies zeigt, dass der erfindungsgemässe Wirkstoff die Haut durchdringt und an den gewünschten Stellen in der Haut seine Wirkung entfaltet. Damit stehen diese Beobachtungen mit den Ergebnissen in Einklang, die bei der Behandlung von übermässigem Haarfetten, androgenetischer Alopezie und Akne vulgaris erhalten wurden.

Die Untersuchungsergebnisse zeigen, dass der erfindungsgemäss eingesetzte 11α-Hydroxyprogesteron-Wirkstoff ein Haar- und Hautpflegemittel ist, das seine kosmetische Wirkung insbesondere bei androgenetisch bedingten Störungen des Kopfhaares sowie bei Akne vulgaris durch lokale Anwendung entfaltet, ohne dass hormonale oder sonstige lokale oder allgemeine Nebenwirkungen auftreten. Es erfüllt daher alle Erfordernisse, die an ein typisches Schönheitspflegemittel gestellt werden.

Die Erfindung betrifft ferner auch die Verwendung der Wirkstoffe zur Herstellung von kosmetischen Haar- und Hautpflegemitteln.

Die erfindungsgemässen Haar- und Hautpflegemittel enthalten neben dem Wirkstoff die in der Kosmetik für den jeweiligen Zweck üblichen Zusatzstoffe. Auf diese Weise können die erfindungsgemässen Mittel vorzugsweise als Haarwasser, Haaröl, Hautfunktionsöl, Hautnähremulsion, Aerosolspray, Shampoo oder Frisiermittel angewandt werden. Aber auch andere allgemeine und spezifische haut- und haarpflegende Zubereitungen können die erfindungsgemäss eingesetzten Wirkstoffe enthalten.

Der Wirkstoff wird diesen Zubereitungen in einer Menge zugemischt, die eine zuverlässige Wirkung bei regelmässiger Anwendung gewährleistet. Versuche haben gezeigt, dass dies der Fall ist, wenn der Wirkstoff in den kosmetischen Mitteln in einer Konzentration von ewa 0,5 bis 2,0 Gew.-%, insbesondere von 0,8 bis 1,4 Gew.-%, bezogen auf die Gesamtmenge des Kosmetikums, vorliegt.

Die Erfindung wird anhand der folgenden Beispiele für die Verwendung von 11α-Hydroxyprogesteron bzw. eines seiner pharmazeutisch verträglichen Ester weiter erläutert.

Beispiel 1:
Herstellung eines Haarwassers

| Formulierung: | |
|---|---|
| Isopropylalkohol extra | 52 g |
| Ethylpolyglykolacetat | 2 g |
| 11α-Hydroxyprogesteron | 1,2 g |
| Parfümöl | 0,8 g |
| dest. Wasser | 44,0 g |
| | 100,0 g |

Der Wirkstoff, das Parfümöl und das Ethylpolyglykolacetat werden mit dem Isopropylalkohol vermischt und das Gemisch mit Wasser auf 100 g aufgefüllt.

Beispiel 2:
Herstellung eines Haaröls

| | |
|---|---|
| Isopropylpalmitat | 30,0 g |
| Isopropylmyristat | 20,0 g |
| Vaselinöl | 47,0 g |
| 11α-Acetoxyprogesteron | 1,8 g |
| Parfümöl | 0,7 g |
| Dichlorophen | 0,5 g |
| | 100,0 g |

Beispiel 3:
Herstellung einer Hautcreme

| | |
|---|---|
| Mandelöl (fett) | 10,0 g |
| Cetiol (Gemisch aus Estern ungesättigter $C_{12}$–$C_{18}$-Fettsäuren) | 12,0 g |
| Cetyl- und Stearylalkohol | 12,0 g |
| Na-Salz des Cetyl-stearylschwefelsäureesters | 3,0 g |
| Glycerin | 6,0 g |
| 11α-Acetoxyprogesteron | 1,4 g |
| Parfümöl | 0,6 g |
| Wasser | 55,0 g |
| | 100,0 g |

Beispiel 4:
Herstellung eines Shampoos

| | |
|---|---|
| Fettsäure-Eiweiss-Kondensationsprodukt | 25,0 g |
| Glycerinmonolaurat-monosulfat | 20,0 g |
| Glycerin | 7,0 g |
| 11α-Hydroxyprogesteron | 0,5 g |
| Parfümöl | 0,5 g |
| Wasser | 47,0 g |
| | 100,0 g |

**Patentansprüche**

1. Kosmetisches Haar- und Hautpflegemittel, gekennzeichnet durch einen Gehalt an 11 α-Hydroxyprogesteron und/oder einem pharmazeutisch verträglichen Ester davon als Wirkstoff im Gemisch mit den in der Kosmetik für den jeweiligen Zweck üblichen Zusatzstoffen.

2. Mittel nach Anspruch 1, dadurch gekennzeichnet, dass die Zusatzstoffe ein Haarwasser oder Haaröl darstellen.

3. Mittel nach Anspruch 1, dadurch gekennzeichnet, dass die Zusatzstoffe eine Hautcreme darstellen.

4. Mittel nach Anspruch 1, dadurch gekennzeichnet, dass die Zusatzstoffe ein Hautfunktionsöl oder eine Hautnähremulsion darstellen.

5. Mittel nach Anspruch 1, dadurch gekennzeichnet, dass die Zusatzstoffe einen Aerosolspray darstellen.

6. Mittel nach Anspruch 1, dadurch gekennzeichnet, dass die Zusatzstoffe ein Shampoo darstellen.

7. Mittel nach Anspruch 1, dadurch gekennzeichnet, dass die Zusatzstoffe ein Frisiermittel darstellen.

8. Mittel nach einem der vorhergehenden Ansprüche, dadurch gekennzeichnet, dass der Wirkstoff in einer Konzentration von etwa 0,5 bis 2,0 Gewichtsprozent, insbesondere von 0,8 bis 1,4 Gewichtsprozent, bezogen auf die Gesamtmenge des Kosmetikums, vorliegt.

9. Mittel nach einem der vorherigen Ansprüche, dadurch gekennzeichnet, dass es als Wirkstoff 11α-Acetoxy-progesteron enthält.

10. Verwendung von 11α-Hydroxy-progesteron und/oder einem seiner pharmazeutisch verträglichen Ester zur Herstellung eines kosmetischen Haar- bzw. Hautpflegemittels gemäss einem der Ansprüche 1 bis 9.

**Revendications**

1. Produit de beauté cosmétique pour la chevelure et la peau, caractérisé en ce qu'il renferme de la 11α-hydroxyprogestérone et/ou un de ses esters pharmaceutiquement acceptables, en tant que composé actif, en mélange avec les constituants classiques utilisés en cosmétique.

2. Produit selon la revendication 1, caractérisé en ce que lesdits constituants forment une lotion capillaire ou une brillantine.

3. Produit selon la revendication 1, caractérisé en ce que lesdits constituants forment une crème pour la peau.

4. Produit selon la revendication 1, caractérisé en ce que lesdits constituants forment une huile fonctionnelle pour la peau ou une émulsion nourrissante pour la peau.

5. Produit selon la revendication 1, caractérisé en ce que lesdits constituants forment une pulvérisation en aérosol.

6. Produit selon la revendication 1, caractérisé en ce que lesdits constituants forment un shampooing.

7. Produit selon la revendication 1, caractérisé en ce que lesdits constituants forment un produit capillaire.

8. Produit selon la revendication 1 à 7, caractérisé en ce que le composé actif est présent à une concentration pondérale comprise entre 0,5 et 2% environ et plus particulièrement entre 0,8 et 1,4% par rapport au poids total de la composition cosmétique.

9. Produit selon une quelconque des revendications 1 à 8, caractérisé en ce qu'il renferme la 11α-hydroxyprogestérone en tant que composé actif.

10. Utilisation de la 11α-hydroxyprogestérone et/ou un de ses esters pharmaceutiquement acceptable pour préparer un produit de beauté cosmétique pour la chevelure ou la peau selon une quelconque des revendications 1 à 9.

**Claims:**

1. Cosmetic hair or skin composition characterized by a content of 11α-hydroxyprogesterone

and/or a pharmaceutically compatible ester thereof as active ingredient in admixture with additives being used within the cosmetic field for the respective purpose.

2. Composition according to claim 1, characterized in that the additives are a hair tonic or a hair oil.

3. Composition according to claim 1, characterized in that the additives are a skin cream.

4. Composition according to claim 1, characterized in that the additives are a skin function oil or a nutrient skin emulsion.

5. Composition according to claim 1, characterized in that the additives are an aerosol spray.

6. Composition according to claim 1, characterized in that the additives are a shampoo.

7. Composition according to claim 1, characterized in that the additives are a hair dressing agent.

8. Composition according to one of the former claims characterized in that the active ingredient is present in a concentration of about 0.5 to 2.0% by weight and preferably of 0.8 to 1.4% by weight based on the total weight of the cosmetic composition.

9. Composition according to one of the former claims, characterized in that it contains as active ingredient 11α-acetoxyprogesterone.

10. Use of 11α-hydroxyprogesterone and/or of a pharmaceutically compatible ester thereof for the preparation of cosmetic hair treating compositions or skin treating compositions according to one of the claims 1 to 9.